# FASCICULE DE BREVET EUROPEEN

(11) **EP 0 810 292 B1**
(45) Date de publication et mention de la délivrance du brevet: **23.01.2002**
(21) Numéro de dépôt: 97401148.8
(22) Date de dépôt: 26.05.1997
(51) Int. Cl.: C13K 1/00, C13K 13/00, C12P 19/02, C07C 51/31, C07C 51/285, C07C 59/105, C12P 7/58, C07H 3/02, C07C 29/141, C07C 31/18

(54) **Procédé de préparation du d-arabitol**
Verfahren zur Herstellung von D-Arabit
Process for the preparation of D-arabitol

(30) Priorité: 29.05.1996 FR 9606599
(43) Date de publication de la demande: 03.12.1997
(73) Titulaire: Roquette Frères, 62136 Lestrem (FR)
(72) Inventeur: Delobeau, Didier, 59660 Merville (FR); Moine, Didier, 59660 Merville (FR)
(74) Mandataire: Boulinguiez, Didier

(56) Documents cités:
- EP-A- 0 142 725
- WO-A-93/19030
- FR-A- 2 453 214
- US-A- 4 156 076
- US-A- 4 259 443
- US-A- 4 845 208
- US-A- 5 132 452
- JOURNAL OF THE AMERICAN CHEMICAL SOCIETY, vol. 72, no. 10, 16 Octobre 1950, WASHINGTON, DC, US, page 4546 XP002024334 H.G. FLETCHER, JR., ET AL.: "Improvements in the preparation of D-arabinose from calcium D-gluconate and of D-lyxose from calcium D-galactonate"
- PATENT ABSTRACTS OF JAPAN vol. 006, no. 127 (C-113), 13 Juillet 1982 & JP 57 054198 A (TOWA KASEI KOGYO), 31 Mars 1982,
- JOURNAL OF THE AMERICAN CHEMICAL SOCIETY, vol. 56, no. 197, 5 Juillet 1934, WASHINGTON, DC, US, pages 1632-1633, XP002024368 R.C. LOCKETT, ET AL.: "Improvements in the preparation of d-arabinose from calcium gluconate"
- BERICHTE DER DEUTSCHEN CHEMISCHEN GESELLSCHAFT, vol. 32, 27 Février 1899, WEINHEIM, DE, pages 550-560, XP002024369 O. RUFF: "d- und r-Arabinose"

## Description

La présente invention a pour objet un procédé de préparation du D-arabitol à partir de mélanges de glucose et de galactose.

Avantageusement la présente invention concerne un procédé de préparation du D-arabitol à partir d'hydrolysats de lactose.

Le D-arabitol est un intermédiaire de synthèse important pour la fabrication du xylitol. Le xylitol est un substitut acariogène du saccharose et en tant que tel, il connaît un succès croissant, notamment dans les confiseries sans sucres.

Des procédés permettant d'obtenir du D-arabitol et de transformer le D-arabitol en xylitol sont par exemple décrits dans les brevets américains US 5.096.820 et US 5.238.826 dont la Demanderesse est cessionnaire.

Ces procédés consistent à oxyder par voie microbienne, le D-arabitol, en D-xylulose, à isomériser ce D-xylulose en D-xylose, puis à hydrogéner catalytiquement le D-xylose en xylitol.

La matière première permettant d'obtenir le D-arabitol est dans ce cas le glucose qui est, soit fermenté directement en D-arabitol, soit oxydé en acide gluconique qui est lui-même décarboxylé en D-arabinose lequel est enfin hydrogéné catalytiquement en D-arabitol.

De tels procédés souffrent de l'inconvénient de n'utiliser que le glucose comme matière première.

Un autre procédé permettant d'obtenir le D-arabitol puis de transformer le D-arabitol en xylitol est aussi décrit dans la demande internationale de brevet 93/19030.

Il consiste à isomériser à haute température, sous forte pression d'hydrogène et sous l'action d'un catalyseur à base de ruthénium, une solution de D-arabitol. Environ 20 % de l'arabitol est ainsi isomérisé en xylitol.

Le xylitol est ensuite extrait de ce mélange par chromatographie et le D-arabitol qui n'a pas réagi est soumis à nouveau à l'isomérisation.

La matière première permettant d'obtenir le D-arabitol est dans ce cas, soit le glucose qui est dégradé oxydativement en acide D-arabinonique lequel est hydrogéné catalytiquement en D-arabitol après lactonisation de l'acide, soit le galactose qui est dégradé oxydativement en acide D-lyxonique lequel est hydrogéné catalytiquement en D-arabitol, également après lactonisation de l'acide.

Un tel procédé possèderait donc l'avantage de pouvoir utiliser les hydrolysats de lactose comme matière première de la fabrication de D-arabitol mais souffre de l'énorme inconvénient que l'hydrogénation catalytique des acides D-arabinonique ou D-lyxonique en D-arabitol s'effectue extrêmement mal et avec de très mauvais rendements.

La raison en est, comme indiqué dans cette demande de brevet, que les sels de ces acides pentoniques ne s'hydrogènent pas et qu'il faut d'abord transformer ceux-ci en esters ou en lactones.

Cependant, même en utilisant les catalyseurs au ruthénium très onéreux, les rendements d'hydrogénation des lactones sont très mauvais. L'emploi de catalyseurs plus classiques, à base de nickel de RANEY, ne permet pas non plus, même en les employant en poids pour poids par rapport à la D-arabino-1-4-lactone soumise à l'hydrogénation, d'obtenir de meilleurs rendements.

Un autre procédé d'obtention du D-arabitol à partir du lactose est décrit dans le brevet américain US 4.156.076.

Le procédé décrit dans ce brevet consiste à oxyder le lactose en acide lactobionique, puis à hydrolyser cet acide en un mélange de galactose et d'acide gluconique. Le sucre et l'acide sont alors séparés et seul l'acide gluconique séparé est utilisé pour obtenir le D-arabitol par décarboxylation en D-arabinose puis hydrogénation. La moitié de la matière première est ainsi perdue sous forme de galactose ou doit être valorisée autrement.

Tous ces procédés d'obtention du D-arabitol ne donnent donc pas satisfaction car pour les premiers cités, ils souffrent de l'inconvénient d'utiliser la matière première relativement onéreuse qu'est le glucose et pour les seconds d'entre eux, ils souffrent des mauvais rendements réactionnels qui viennent lourdement grever le prix extrêmement intéressant du lactose, lié à sa grande disponibilité et à la difficulté que l'on a à le valoriser sous une autre forme.

L'objet de la présente invention est donc de fournir un procédé permettant de pallier les inconvénients précités et de trouver un débouché valorisant au lactose en le transformant en D-arabitol.

Il a maintenant été trouvé, et c'est ce qui constitue l'objet de la présente invention, un procédé de préparation du D-arabitol caractérisé par le fait qu'il comporte les étapes suivantes :
- l'hydrolyse d'une solution de lactose,
- l'oxydation du mélange de glucose et de galactose ainsi obtenu en un mélange des acides gluconique et galactonique,
- la décarboxylation de ce mélange des acides gluconique et galactonique en un mélange de D-arabinose et de D-lyxose,
- l'hydrogénation catalytique de ce mélange de D-arabinose et de D-lyxose en D-arabitol.

Bien que le lactose soit la matière première préférée dans le procédé de l'invention, des mélanges de glucose et de lactose ou de galactose et de lactose peuvent être aussi utilisés dans ce procédé. De même, certains hydrolysats végétaux de bois ou de paille, constitués presque exclusivement de glucose et de galactose, peuvent aussi être utilisés.

Le procédé de l'invention comporte donc une première opération d'hydrolyse d'une solution de lactose, ce qui permet de scinder en ses deux composants cette molécule et d'obtenir un mélange équimoléculaire de glucose et de galactose libres.

La fonction hémiacétalique du galactose qui était engagée dans une liaison osidique avec le glucose se trouve libérée par cette hydrolyse et devient dès lors disponible pour l'oxydation, au même titre que la fonction hémiacétalique du glucose qui était déjà libre dans la molécule de lactose.

Sans séparation des composants de l'hydrolysat intermédiaire, on enchaîne alors l'étape d'oxydation ménagée du glucose et du galactose, oxydation qui transforme en fonctions carboxyliques les fonctions hémiacétaliques du glucose et du galactose. On obtient ainsi un mélange contenant les acides gluconique et galactonique.

Toujours sans séparer les acides obtenus, on poursuit alors le procédé de l'invention avec l'étape de décarboxylation de ces acides.

Par excision de la fonction carboxylique et réinstauration d'une fonction hémiacétalique sur le squelette carboné ainsi raccourci des acides, on obtient un mélange de D-arabinose et de D-lyxose.

Enfin, par hydrogénation catalytique de ce mélange de D-arabinose et de D-lyxose, et toujours sans séparation des deux composants principaux du mélange, on obtient le D-arabitol à l'état pratiquement pur puisque le D-lyxitol est synonyme de D-arabitol.

Le procédé de l'invention est particulièrement intéressant car il permet d'obtenir le D-arabitol avec un très bon rendement de réaction. En effet, toute la molécule de lactose est utilisée, et ce sans jamais devoir procéder à une quelconque séparation des intermédiaires de synthèse.

Enfin, un autre avantage et non des moindres du procédé de l'invention, est que l'étape d'hydrogénation catalytique qu'il comporte s'effectue sur des fonctions hémiacétaliques très faciles à réduire en alcools primaires, et ce avec des rendements et sélectivité voisins de 100 % et à l'aide de catalyseurs bon marché au nickel de RANEY, employés en quantités réellement catalytiques.

Il n'est donc pas nécessaire d'avoir affaire dans le procédé de l'invention, aux coûteux catalyseurs au ruthénium qui s'empoisonnent en outre à la moindre trace d'acide formique, pas plus qu'il n'est nécessaire de devoir lactoniser les acides qui se forment intermédiairement ni d'utiliser de solvants toxiques et dangereux comme indiqué dans la demande internationale de brevet WO 93/19030 déjà citée.

Le procédé de l'invention permet ainsi de valoriser au meilleur coût toute la molécule de lactose dans un procédé performant: de préparation du D-arabitol.

Conformément au procédé de l'invention, l'étape d'hydrolyse du lactose est conduite par voie chimique ou enzymatique.

Lorsque l'on emploie la voie chimique, on préfère utiliser des acides forts et non oxydants dans les conditions de leur utilisation. C'est ainsi que les acides chlorhydrique ou sulfurique sont préférés tandis que l'acide nitrique est à éviter puisqu'il conduit à des réactions plus complexes d'oxydation du lactose. Les quantités d'acide fort à utiliser sont variables mais demeurent catalytiques. Elles sont fonction de la durée de l'hydrolyse, de la température de cette hydrolyse et de la concentration en lactose de la solution à hydrolyser.

L'hydrolyse par voie enzymatique du lactose peut être réalisée à l'aide de béta-galactosidase. Les températures employées dans le cas de l'hydrolyse enzymatique sont plus faibles que celles que l'on emploie lorsque l'on procède à l'hydrolyse par voie chimique du lactose. On pourra donc être amené à employer des concentrations plus faibles en lactose pour prévenir toute cristallisation intempestive de ce sucre.

Une enzyme particulièrement adaptée est la béta-galactosidase LACTOZYM^{R} commercialisée par NOVO.

Que l'on procède par voie chimique ou par voie enzymatique, l'hydrolyse peut être conduite de façon discontinue ou continue mais on préférera dans tous les cas, ajuster les différents paramètres : durée, concentration du catalyseur ou de l'enzyme, température, concentration du lactose, de manière à obtenir un taux d'hydrolyse du lactose au moins supérieur à 90 %, de préférence supérieur à 95 %.

De tels ajustements des conditions d'hydrolyse sont parfaitement à la portée de l'homme de l'art qui pourra mesurer le taux d'hydrolyse de façon simple par le dosage des extrêmités réductrices qui apparaissent, selon la méthode de BERTRAND par exemple pour ne citer que cette méthode.

Selon le procédé de l'invention, on n'isole pas les sucres obtenus et l'on enchaîne directement l'étape suivante d'oxydation du mélange de glucose et de galactose qui se forment, en un mélange des acides gluconique et galactonique.

Conformément au procédé de l'invention, cette étape d'oxydation peut être conduite soit par voie chimique, soit par voie microbiologique.

La voie chimique préférée dans le procédé de l'invention consiste à oxyder à l'aide d'air ou d'oxygène en milieu alcalin et à l'aide de catalyseurs au palladium, le mélange de glucose et de galactose obtenu à l'étape précédente.

Un procédé tout particulièrement préféré est celui qui a été décrit dans le brevet américain US 4.845.208 dont la Demanderesse est aussi cessionnaire et qui consiste à utiliser comme catalyseur d'oxydation, du palladium fixé sur charbon actif et dopé au bismuth.

On peut également envisager dans le procédé de l'invention, d'oxyder les mélanges de glucose et de galactose par voie électrolytique ou à l'aide d'hypobromite.

Lorsque l'on emploie le procédé décrit dans le brevet américain US 4.845.208, on préfère travailler avec une concentration en sucres comprise entre 10 et 40 %, à une température comprise entre 25 et 60°C avec une quantité de palladium exprimée en termes de métal, comprise entre 0,01 % et 0,4 % du poids des sucres. Dans des conditions où l'apport d'oxygène n'est pas limitant, l'oxydation du mélange de glucose et de galactose est complète en environ 30 minutes à 5 heures.

L'agent alcalin préféré dans le cadre de la présente invention est le carbonate ou l'hydroxyde de calcium. Il est employé pour maintenir un pH constant au cours de la réaction d'oxydation, de préférence compris entre 8.0 et 10.0.

On peut aussi procéder à l'oxydation du mélange de glucose et de galactose par voie microbienne. Dans ce cas, on ajoute aux solutions aqueuses de l'hydrolysat de lactose de petites quantités de sels minéraux et d'éléments nutritifs que l'on apporte commodément sous forme de liqueur de macération du maïs ou d'extrait de levure par exemple.

Après stérilisation du milieu de culture ainsi obtenu, on ensemence celui-ci avec une culture bactérienne d'un microorganisme capable d'oxyder à la fois le glucose en acide gluconique et le galactose en acide galactonique.

Des microorganismes tels que les gluconobacter oxydans ATCC 19357 ou encore ATCC 23773 sont parfaitement capables d'oxyder ces deux sucres en les acides aldoniques correspondants.

Lorsque l'on emploie ces microorganismes, on préfère travailler à des concentrations en sucres comprises entre 100 et 250 grammes par litre et à une température de 25 à 35°C.

Dans des conditions où l'apport d'oxygène au moût de culture n'est pas un facteur limitant, l'oxydation du mélange de glucose et de galactose est complète en environ 15 à 30 heures.

Il faut veiller, pour que l'oxydation bactérienne se déroule dans des conditions satisfaisantes, à maintenir le pH à une valeur comprise entre 4,0 et 7,0. L'agent alcalin préféré dans le cadre de la présente invention, est aussi le carbonate ou l'hydroxyde de calcium.

Que l'on procède à l'oxydation de l'hydrolysat de lactose par voie chimique ou microbienne, on préfère mener celle-ci jusqu'à quasi-disparition des sucres réducteurs et notamment jusqu'à ce que ces sucres réducteurs représentent moins de 10 % et encore plus préférentiellement moins de 5 % de la matière sèche soumise à l'oxydation.

Au terme de cette étape d'oxydation, on filtre les milieux réactionnels à une température suffisante pour maintenir en solution les sels de calcium des acides gluconique et galactonique afin d'en éliminer, soit le catalyseur, soit les microorganismes.

A la connaissance de la Demanderesse, une telle étape d'oxydation simultanée d'un mélange de galactose et de glucose n'a jamais été décrite.

Selon le procédé de l'invention, on ne sépare pas les sels de calcium des acides gluconique et galactonique ainsi obtenus et on enchaîne directement l'étape de décarboxylation de ces acides sur ce mélange.

Bien que cette étape de décarboxylation puisse être menée à l'aide d'acide hypochloreux, le procédé de l'invention préfère mettre en oeuvre la méthode de RUFF qui utilise le peroxyde d'hydrogène.

A la connaissance de la Demanderesse, une telle étape de décarboxylation, bien que déjà décrite, tant pour le gluconate de calcium que pour le galactonate de calcium (FLETCHER H.G. et Coll. Journal of the American Chemical Society, 1950, Vol. 72, P. 4546) n'a cependant jamais été décrite pour un mélange de ces deux sels.

La raison pour laquelle le procédé de l'invention se satisfait le mieux de l'utilisation des sels de calcium des acides gluconique et galactonique et du procédé de RUFF, est que cette étape de décarboxylation de ces acides hexoniques conduisant aux pentoses correspondants, mène ainsi au carbonate de calcium très peu soluble. On peut donc par simple filtration du carbonate de calcium, abaisser fortement la charge minérale de la solution de pentoses résultante.

L'utilisation d'autres sels que les sels de calcium est théoriquement possible mais s'avère nettement plus onéreuse et moins pratique.

De même, l'utilisation d'hypochlorite de sodium en lieu et place du peroxyde d'hydrogène utilisé dans le procédé de RUFF alourdirait aussi largement cette charge minérale.

Selon un mode préféré du procédé de l'invention, l'étape de décarboxylation est donc menée sur une solution des sels de calcium des acides gluconique et galactonique.

On préfère opérer sur des solutions contenant de 100 à 400 grammes par litre et de préférence de 200 à 300 grammes par litre d'hexonates de calcium anhydres et à une température d'environ 30 à 50°C.

Cette réaction de décarboxylation est catalysée par les ions ferriques. Dans le procédé de l'invention on préfère ajouter à la solution d'hexonates de calcium, du sulfate ferrique à raison de 1 à 5 % de sulfate ferrique anhydre par rapport au poids d'hexonates anhydres. Ces ions ferriques peuvent cependant être amenés sous une autre forme.

Sous agitation, on ajoute alors lentement, le peroxyde d'hydrogène, de préférence sous forme d'eau oxygénée d'une richesse de 30 %, à raison de 120 à 140 millilitres environ pour 100 grammes d'hexonates anhydres.

L'addition de l'eau oxygénée est effectuée à un débit tel que la température du milieu réactionnel ne s'élève pas au-delà de 50°C. La réaction est généralement achevée en 2 à 8 heures et son terme est marqué par l'apparition d'une couleur pourpre.

L'abondant précipité de carbonate de calcium qui se forme est filtré, de préférence après avoir laissé le milieu réactionnel se refroidir. On déminéralise ensuite ce milieu réactionnel sur une batterie de résines échangeuses d'ions constituée d'une résine cationique forte et d'une résine anionique faible, résines auxquelles on peut adjoindre une résine cationique faible et anionique forte et/ou un lit mélangé de résines cationique et anionique fortes.

La charge minérale des milieux réactionnels filtrés peut aussi être abaissée par des techniques de chromatographie d'exclusion d'ions sur des résines cationiques chargées sous forme de calcium avant de procéder à l'étape de déminéralisation complète effectuée avec les résines citées précédemment.

Après ces traitements de purification qui peuvent également comprendre une étape de décoloration à l'aide de charbon végétal, on obtient une solution incolore contenant quasi-exclusivement du D-arabinose et du D-lyxose.

La dernière étape du procédé selon l'invention, consiste à hydrogéner catalytiquement cette solution de D-arabinose et de D-lyxose sans en séparer ces deux sucres.

Comme il a déjà été dit plus avant, une telle hydrogénation portant sur des fonctions hémiacétaliques est extrêmement facile à réaliser. C'est là un autre des gros avantages du procédé de l'invention par rapport au procédé exposé dans la demande internationale de brevet WO 93/19030 où l'hydrogénation porte sur des fonctions carboxyliques estérifiées des acides D-arabinonique et D-lyxonique.

A la connaissance de la Demanderesse, une telle hydrogénation d'un mélange grossièrement équimoléculaire de D-arabinose et de D-lyxose est également nouvelle.

L'hydrogénation d'un tel mélange de sucres s'effectue cependant conformément aux règles de l'art qui conduisent par exemple à la production du sorbitol à partir du glucose.

On peut utiliser pour cette étape aussi bien des catalyseurs à base de ruthénium que des catalyseurs au nickel de RANEY.

On préfère cependant utiliser des catalyseurs au nickel de RANEY qui sont moins onéreux.

Dans la pratique, on utilise de 1 à 10 % en poids de catalyseur par rapport à la matière sèche de sucre soumis à l'hydrogénation. L'hydrogénation s'effectue de préférence sur des sirops dont la matière sèche est comprise entre 15 et 50 %, dans la pratique voisine de 30 à 45 %, sous une pression d'hydrogène comprise entre 20 et 200 bars. Elle peut être effectuée de manière continue ou discontinue.

Lorsque l'on opère de manière discontinue, la pression d'hydrogène utilisée est généralement comprise entre 30 et 60 bars et la température à laquelle se déroule l'hydrogénation est comprise entre 100 et 150°C. On veille aussi à maintenir le pH du milieu d'hydrogénation par l'addition de soude ou de carbonate de soude par exemple, mais sans dépasser un pH de 9,0. Cette manière de faire permet d'éviter l'apparition d'autres pentitols tels que le xylitol ou le ribitol qui peuvent apparaître consécutivement à une isomérisation alcaline du D-lyxose ou du D-arabinose. On arrête la réaction lorsque la teneur du milieu réactionnel en sucres réducteurs est devenue inférieure à 1 %, de préférence encore inférieure à 0,5 %.

Après refroidissement du milieu réactionnel, on élimine le catalyseur par filtration et on déminéralise le sirop de D-arabitol ainsi obtenu sur des résines cationiques et anioniques.

A ce stade, les sirops contiennent au moins 90 % de D-arabitol et il est aisé d'en purifier celui-ci par cristallisation après concentration et refroidissement des solutions.

Toutefois la pureté des solutions de D-arabitol obtenues par le procédé de l'invention fait qu'une telle purification n'est généralement pas nécessaire lorsque l'on veut poursuivre la transformation du D-arabitol en xylitol par les procédés décrits dans les brevets américains US 5.096.820 ou US 5.238.826 ou encore dans la demande internationale de brevet 93/19030.

Ainsi donc le procédé de la présente invention permet, par la combinaison particulière des étapes qu'il revendique, connues dans leur principe mais jamais décrites dans cet ordre d'enchaînement sur les mélanges de sucres ou d'acides aldoniques qui sont concernés, de valoriser facilement et économiquement le lactose en le transformant en D-arabitol.

Etant donné qu'aucun intermédiaire de synthèse ne nécessite d'être isolé dans le procédé de cette invention, le D-arabitol est obtenu avec un rendement excellent, voisin de 50 % du lactose mis en oeuvre.

L'invention sera mieux comprise au moyen de l'exemple qui suit et qui a pour seul but de mieux illustrer l'invention.

### EXEMPLE :

### 1/ HYDROLYSE DU LACTOSE

On met en solution dans de l'eau à une matière sèche de 15 % du lactose cristallisé sous sa forme commerciale de cristaux monohydratés.

On porte cette solution de lactose à la température de 40°C et on ajuste son pH à 6,5.

On ajoute alors 12.000 unités LAU de béta-galactosidase LACTOZYM^{R} 3000 L commercialisée par NOVO NORDISK DANEMARK pour cent grammes de matière sèche de lactose, ce qui représente 4 ml d'enzyme et on laisse l'hydrolyse se poursuivre durant 8 heures.

Le taux d'hydrolyse du lactose est alors de 95 %.

On porte ensuite la solution à l'ébullition pour dénaturer l'enzyme puis on procède à sa purification par décoloration et déminéralisation.

### 2/ OXYDATION EN ACIDES GLUCONIQUE ET GALACTONIQUE

On prépare un catalyseur constitué de charbon actif sur lequel on a déposé successivement du palladium puis du bismuth à raison de 35 % de bismuth par rapport au palladium ainsi qu'il est indiqué à l'exemple 4 du brevet américain US 4.845.208.

On procède ensuite à l'oxydation de l'hydrolysat de lactose dont la matière sèche a été abaissée à 12 % par suite des dilutions causées par sa purification.

Pour cela, on procède dans une cuve aérée, agitée et thermostatée à 35°C, munie d'une sonde de mesure du pH, à l'aide de 3 % de catalyseur obtenu comme indiqué par rapport à la matière sèche d'hydrolysat de lactose.

Tout au long de la réaction qui se déroule en 3h30, on maintient le pH du milieu réactionnel à une valeur comprise entre 8,5 et 9,1 par addition progressive d'un lait de chaux.

A la fin de la réaction qui se traduit par un arrêt de la consommation de la chaux, on filtre le catalyseur. La teneur en sucres réducteurs de ce milieu réactionnel est de 1,5 %.

### 3/ DECARBOXYLATION EN D-ARABINOSE ET D-LYXOSE

Au filtrat des sels de calcium des acides gluconique et galactonique ainsi obtenu, on ajoute une solution de sulfate ferrique de manière à obtenir une concentration de 3 % de sulfate ferrique anhydre par rapport à la matière sèche des hexonates de calcium mis en oeuvre.

On agite et l'on thermostate cette solution à 40°C puis on y ajoute lentement de l'eau oxygénée d'une concentration de 30 % jusqu'à l'obtention d'une couleur brun-pourpre persistante du milieu réactionnel. Cette addition de l'eau oxygénée a été effectuée en 6 heures et on a dû ajouter 130 millilitres d'eau oxygénée pour 100 grammes d'hexonates de calcium mis en oeuvre.

Après refroidissement durant quelques heures de ce milieu réactionnel, on filtre le carbonate de calcium puis on déminéralise ce filtrat sur une résine cationique forte régénérée sous forme hydrogène puis une résine anionique faible régénérée sous forme hydroxyle.

On obtient ainsi une solution de D-arabinose et de D-lyxose, virtuellement incolore que l'on concentre à 40 % de matières sèches.

### 4/ HYDROGENATION EN D-ARABITOL

Dans un réacteur agité et thermostaté, on introduit ce sirop de pentoses auquel on a ajouté 5 % de catalyseur au nickel de RANEY puis on soumet ce réacteur à une pression d'hydrogène de 50 bars et on le chauffe à 120°C.

On maintient au cours de cette hydrogénation, le pH au voisinage de la neutralité à l'aide d'une solution de carbonate de soude. Après 5 heures, la teneur en sucres réducteurs du milieu réactionnel est devenue égale à 0,15 % et l'on refroidit alors le réacteur.

On filtre le catalyseur puis l'on déminéralise sur résines cationique et anionique fortes le sirop hydrogéné.

On obtient un sirop incolore contenant 92 % de D-arabitol.

## Revendications

1. Procédé de préparation du D-arabitol **caractérisé par le fait qu'**il comporte les étapes suivantes :
- l'hydrolyse d'une solution de lactose,
- l'oxydation du mélange de glucose et de galactose ainsi obtenu en un mélange des acides gluconique et galactonique,
- la décarboxylation de ce mélange des acides gluconique et galactonique en un mélange de D-arabinose et de D-lyxose,
- l'hydrogénation catalytique de ce mélange de D-arabinose et de D-lyxose en D-arabitol.

2. Procédé de préparation du D-arabitol selon la revendication 1 **caractérisé par le fait que** l'hydrolyse de la solution de lactose est effectuée par voie chimique.

3. Procédé de préparation du D-arabitol selon la revendication 1 **caractérisé par le fait que** l'hydrolyse de la solution de lactose est effectuée par voie enzymatique.

4. Procédé de préparation du D-arabitol selon les revendications 1 à 3 **caractérisé par le fait que** l'oxydation du mélange de glucose et de galactose est effectuée à l'aide d'air ou d'oxygène, en milieu alcalin, et à l'aide de catalyseurs au palladium.

5. Procédé de préparation du D-arabitol selon les revendications 1 à 3 **caractérisé par le fait que** l'oxydation du mélange de glucose et de galactose est effectuée par voie microbienne à l'aide de microorganismes du genre gluconobacter oxydans.

6. Procédé de fabrication du D-arabitol selon les revendications 1 à 5 **caractérisé par le fait que** les acides gluconique et galactonique sont neutralisés sous forme de sels de calcium.

7. Procédé de fabrication du D-arabitol selon les revendications 1 à 6 **caractérisé par le fait que** la décarboxylation du mélange des acides gluconique et galactonique est réalisée à l'aide de peroxyde d'hydrogène.

8. Procédé de fabrication du D-arabitol selon les revendications 1 à 7 **caractérisé par le fait que** l'hydrogénation catalytique du mélange de D-arabinose et de D-lyxose est réalisée à l'aide de nickel de RANEY.

9. Procédé de fabrication de xylitol, **caractérisé en ce que** l'on prépare du D-arabitol selon le procédé conforme à l'une quelconque des revendications 1 à 8, et **en ce qu'**on le transforme en xylitol.

## Patentansprüche

1. Verfahren zur Herstellung von D-Arabit, **dadurch gekennzeichnet, dass** es die folgenden Schritte umfasst:
- Hydrolyse einer Lactoselösung,
- Oxidation der auf diese Weise erhaltenen Mischung von Glucose und Galactose zu einer Mischung von Gluconsäure und Galactonsäure,
- Decarboxylierung dieser Mischung von Gluconsäure und Galactonsäure zu einer Mischung von D-Arabinose und D-Lyxose,
- katalytische Hydrierung dieser Mischung von D-Arabinose und D-Lyxose zu D-Arabit.

2. Verfahren zur Herstellung von D-Arabit nach Anspruch 1, **dadurch gekennzeichnet, dass** die Hydrolyse der Lactoselösung auf chemischem Weg durchgeführt wird.

3. Verfahren zur Herstellung von D-Arabit nach Anspruch 1, **dadurch gekennzeichnet, dass** die Hydrolyse der Lactoselösung auf enzymatischem Weg durchgeführt wird.

4. Verfahren zur Herstellung von D-Arabit nach den Ansprüchen 1 bis 3, **dadurch gekennzeichnet, dass** die Oxidation der Mischung von Glucose und Galactose mit Hilfe von Luft oder Sauerstoff in alkalischem Medium und mit Hilfe von Palladiumkatalysatoren durchgeführt wird.

5. Verfahren zur Herstellung von D-Arabit nach den Ansprüchen 1 bis 3, **dadurch gekennzeichnet, dass** die Oxidation der Mischung von Glucose und Galactose auf mikrobiellem Weg mit Hilfe von Mikroorganismen der Gattung Gluconobacter oxydans durchgeführt wird.

6. Verfahren zur Herstellung von D-Arabit nach den Ansprüchen 1 bis 5, **dadurch gekennzeichnet, dass** die Gluconsäure und die Galactonsäure in Form von Calciumsalzen neutralisiert werden.

7. Verfahren zur Herstellung von D-Arabit nach den Ansprüchen 1 bis 6, **dadurch gekennzeichnet, dass** die Decarboxylierung der Mischung von Gluconsäure und Galactonsäure mit Hilfe von Wasserstoffperoxid durchgeführt wird.

8. Verfahren zur Herstellung von D-Arabit nach den Ansprüchen 1 bis 7, **dadurch gekennzeichnet, dass** die katalytische Hydrierung der Mischung von D-Arabinose und D-Lyxose mit Hilfe von Raneynickel durchgeführt wird.

9. Verfahren zur Herstellung von Xylit, **dadurch gekennzeichnet, dass** man D-Arabit gemäß dem Verfahren nach einem der Ansprüche 1 bis 8 herstellt und dass man es in Xylit umwandelt.

## Claims

1. A process for the preparation of D-arabitol, **characterized in that** it comprises the following stages:
- hydrolysis of a lactose solution,
- oxidation of the mixture of glucose and galactose thus obtained to a mixture of gluconic and galactonic acids,
- decarboxylation of this mixture of gluconic and galactonic acids to a mixture of D-arabinose and D-lyxose,
- catalytic hydrogenation of this mixture of D-arabinose and D-lyxose to D-arabitol.

2. A process for the preparation of D-arabitol according to claim 1, **characterized in that** the hydrolysis of the lactose solution is carried out by chemical means.

3. A process for the preparation of D-arabitol according to claim 1, **characterized in that** the hydrolysis of the lactose solution is carried out with enzymes.

4. A process for the preparation of D-arabitol according to claims 1-3, **characterized in that** the oxidation of the mixture of glucose and galactose is carried out with the aid of air or oxygen, in an alkaline medium and with the aid of palladium catalysts.

5. A process for the preparation of D-arabitol according to claims 1-3, **characterized in that** the oxidation of the mixture of glucose and galactose is carried out by microbial means with the aid of microorganisms of the gluconobacter oxydans type.

6. A process for the production of D-arabitol according to claims 1-5, **characterizing in that** the gluconic and galactonic acids are neutralised in the form of calcium salts.

7. A process for the production of D-arabitol according to claims 1-6, **characterized in that** the decarboxylation of the mixture of gluconic and galactonic acids is carried out with the aid of hydrogen peroxide.

8. A process for the production of D-arabitol according to claims 1-7, **characterized in that** the catalytic hydrogenation of the mixture of D-arabinose and D-lyxose is carried out with the aid of RANEY nickel.

9. A process for the production of xylitol, **characterized in that** D-arabitol is prepared in accordance with the process according to any one of claims 1-8, and **in that** it is transformed into xylitol.
